# EUROPEAN PATENT APPLICATION

(11) **EP 4 512 909 A1**
(43) Date of publication of application: **26.02.2025**
(21) Application number: 23806817.5
(22) Date of filing: 11.05.2023
(51) Int. Cl.: C12Q 1/6886, C12Q 1/6869, G16B 20/00, G16B 30/00

(54) **USE OF GENE COMBINATION IN PREPARATION OF HUMAN TUMOR HOMOLOGOUS RECOMBINATION DEFICIENCY, TUMOR MUTATION BURDEN AND MICROSATELLITE INSTABILITY GRADING DETECTION PRODUCTS**

(30) Priority: 20.05.2022 CN 202210555507
(71) Applicant: Peking University First Hospital, Beijing 100034 (CN)
(72) Inventor: XIONG, Gengyan, Beijing 100034 (CN); ZHOU, Liqun, Beijing 100034 (CN)
(74) Representative: J A Kemp LLP
(86) International application number: PCT/CN2023/093589
(87) International publication number: WO 2023/221865

(57) **Abstract**

The present application relates to the field of tumor grading detection, and in particular to use of a gene combination in the preparation of a product for human tumor homologous recombination deficiency, tumor mutation burden, and microsatellite instability grading detections. The gene combination consists of a gene set A and a gene fragment set B. The gene combination is obtained from actual high-throughput sequencing data by specific pairwise clustering analysis. Data derived from the real world has higher reliability and credibility. Homologous recombination deficiency, tumor mutation burden, and microsatellite instability grading and prediction can be performed accurately for pan-cancer.

## Description

### CROSS-REFERENCE TO RELATED APPLICATIONS

The present application claims priority to Chinese Patent Application No. 202210555507.8, filed to the China Patent Office on May 20, 2022 and entitled "USE OF GENE COMBINATION IN PREPARATION OF HUMAN TUMOR HOMOLOGOUS RECOMBINATION DEFICIENCY, TUMOR MUTATION BURDEN AND MICROSATELLITE INSTABILITY GRADING DETECTION PRODUCTS", the entire content of which is incorporated herein by reference.

### TECHNICAL FIELD

The present application relates to the field of tumor grading detections, in particular to use of a gene combination in preparation of a product for human tumor homologous recombination deficiency, tumor mutation burden, and microsatellite instability grading detections.

### BACKGROUND

Homologous recombination deficiency (HRD), tumor mutation burden (TMB) and microsatellite instability (MSI) are important indicators commonly used clinically for malignant tumor immunotherapy and judgment on a curative effect of a PARP inhibitor. In general, complete information can be obtained by using different sequencing means respectively for judgment of the above three types of indicators. In general, for the homologous recombination deficiency (HRD) and the microsatellite instability (MSI), a special sequencing panel needs to be designed for performing targeted sequencing on a gene in a specific area, and finally, a final HRD score and MSI score are obtained through calculation for judging whether HRD and MSI are high or low; and for the tumor mutation burden (TMB), whole exome sequencing usually needs to be performed, and whether the TMB is high or low is obtained finally. Though a conventional method for detecting the homologous recombination deficiency (HRD), the tumor mutation burden (TMB) and the microsatellite instability (MSI) is reliable, if the above three indicators are detected at the same time, there are the following significant defects: a detection for the above indicators by using the whole exome sequencing and other methods is high in accuracy but is high in cost, which leads to huge medical cost. Thus, it is urgently necessary to find a novel system for grading the tumor homologous recombination deficiency (HRD), the tumor mutation burden (TMB) and the microsatellite instability (MSI) based on a detection for a specific gene, which may keep accurate on an equivalent level while detection cost is reduced.

### SUMMARY OF THE INVENTION

Thus, a technical problem to be solved by the present application is to provide use of a gene combination in preparation of a product for pan-cancer homologous recombination deficiency (HRD), tumor mutation burden (TMB) and microsatellite instability (MSI) grading detections, which can grade and predict the homologous recombination deficiency (HRD), the tumor mutation burden (TMB) and the microsatellite instability (MSI) ofpan-cancer. For achieving this purpose, the present application adopts a whole exome sequencing technology, sequencing data of a patient in Peking University First Hospital in special screening and grouping is screened, this gene combination is obtained finally for pan-cancer homologous recombination deficiency (HRD), tumor mutation burden (TMB) and microsatellite instability (MSI) grading and prediction, which provides accurate pan-cancer homologous recombination deficiency (HRD), tumor mutation burden (TMB) and microsatellite instability (MSI) grading information and disease prediction information for clinicians and patients, and compared with whole exome sequencing, the present application has obviously lower cost at the same sequencing depth due to fewer involved target genes.

The present application provides use of a gene combination in preparation of a product for human tumor homologous recombination deficiency, tumor mutation burden and/or microsatellite instability grading detections, wherein the gene combination consists of a gene set A and a gene fragment set B;
the gene set A includes at least one of ASAH1, ASXL1, BCOR, BRAF, CALML6, CCDC136, CIDEC, COX18, CSF1R, CYP3A5, DEK, DNMT3A, EGR1, FAM71E2, FGFR1, FKBP7, FLT1, FLT3, FLT4, GNAQ, GLIS1, IDH2, IFITM3, IMMT, KDR, KIT, KMT2A, KNOP1, KRT76, KRT9, KRTAP10-10, KRTAP10-8, MAF, MECOM, MFRP, MLLT3, MNS1, MRTFA, MTOR, MYH11, NF1, NUP214, PDGFRA, PDGFRB, PML, PRB2, PROSER3, RAF1, RARA, RBM15, RET, REXO1, RPN1, RUNX1T1, SCYL1, SLC16A6, SRC, STAG2, TCEAL5, TET2, TMEM82, TP53, TRIM26, U2AF1, U2AF2, UGT1A1, USP35, VEGFA, WBP2NL, WDR44, ZNF20, ZNF700, or ZRSR2;
the gene fragment set B includes at least one of chr2:179479501-179610249, chr2:207989501-208000249, chr2:219719501-219840249, chr2:3679501-3700249, chr3:126249501-126270249, chr3:129319501-129330249, chr3:138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:63179501-63550249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249, or chr22:46309501-47080249; and a position of a gene fragment in the gene fragment set B is annotated by using GRCh37 as a standard, numbers may change in GRCh38 or a new-version human reference genome that emerges in the future, but a position of a directional objective fragment and a gene available for detections do not change.

Optionally, genes included in the gene fragment set B in details are shown in the following table:

**Table 1 Gene fragment set B**

| Position of gene fragment | Genes available for detections |
|---|---|
| chr2:179479501-17961024 9 | at least one of TTN, MIR548N, or LOC100506866 |
| chr2:207989501-20800024 9 | KLF7 |
| chr2:219719501-21984024 9 | at least one of WNT6, CDK5R2, or WNT10A |
| chr2:3679501-3700249 | COLEC11 |
| chr3:126249501-12627024 9 | at least one of C3orf22 or CHST13 |
| chr3:129319501-12933024 9 | PLXND1 |
| chr3:138659501-13877024 9 | at least one of FOXL2, PRR23B, PRR23C, C3 orf72, or PRR23A |
| chr3:183999501-18402024 9 | at least one of PSMD2 or ECE2 |
| chr4:1189501-1230249 | at least one of CTBP1, SPON2, or LOC100130872 |
| chr4:8579501-8590249 | GPR78 |
| chr4:9319501-9330249 | at least one of LOC728369, LOC728373, LOC728379, USP17L5, LOC728393, LOC728400, or LOC728405 |
| chr5:150899501-15094024 9 | FAT2 |
| chr6:147819501-14784024 9 | SAMD5 |
| chr6:157089501-157110249 | at least one of ARID1B or MIR4466 |
| chr6:164889501-16490024 9 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7: 100539501-10056024 9 | ACHE |
| chr8:1939501-1960249 | KBTBD11 |
| chr8:21999501-22070249 | at least one of BMP1, SFTPC, or LGI3 |
| chr8:29189501-29200249 | DUSP4 |
| chr9:91789501-91800249 | SHC3 |
| chr10:99419501-99440249 | at least one of PI4K2A or AVPI1 |
| chr11:17739501-17760249 | at least one of KCNC1 or MYOD1 |
| chr11:63329501-63350249 | at least one of PLA2G16 or PLAAT2 |
| chr12:169501-250249 | at least one of IQSEC3 or LOC574538 |
| chr12:54329501-54350249 | at least one of HOXC12 or HOXC13 |
| chr12:63179501-63550249 | at least one of AVPR1A or PPM1H |
| chr12:7269501-7310249 | at least one of CLSTN3, RBP5, or MATL2963 |
| chr13:114519501-11453024 9 | GAS6 |
| chr15:73649501-73670249 | HCN4 |
| chr15:74209501-74220249 | at least one of LOXL1 or LOC100287616 |
| chr15:78409501-78430249 | CIB2 |
| chr15:83859501-83880249 | HDGFL3 |
| chr18:8809501-8820249 | MTCL1 |
| chr19:24059501-24070249 | ZNF726 |
| chr19:4229501-4250249 | at least one of EBI3 or CCDC94 |
| chr19:46879501-46900249 | PPP5C |
| chr20:22559501-22570249 | FOXA2 |
| chr20:62189501-62200249 | HELZ2 |
| chr21:45949501-46110249 | at least one of TSPEAR, KRTAP12-2, KRTAP12-1, KRTAP10-10, KRTAP10-4, KRTAP10-6, KRTAP10-7, KRTAP10-9, KRTAP10-1, KRTAP10-11, KRTAP10-2, KRTAP10-5, KRTAP10-8, KRTAP10-3, KRTAP12-3, or KRTAP12-4 |
| chr22:19499501-19760249 | at least one of GP1BB, SEPTIN5, TBX1, CLDN5, CDC45, LOC150185, or SEPT5-GP1BB |
| chr22:36649501-38700249 | at least one of hsa-mir-659, CSF2RB, CSNK1E, H1F0, IL2RB, LGALS1, LGALS2, MFNG, MPST, MYH9, NCF4, POLR2F, PVALB, RAC2, SOX10, SSTR3, TST, PLA2G6, GALR3, APOL1, EIF3D, PICK1, CACNG2, IFT27, TRIOBP, CDC42EP1, GCAT, SLC16A8, SH3BP1, MAFF, TXN2, TMEM184B, GGA1, CYTH4, CARD10, EIF3L, PDXP, NOL12, KCTD17, FOXRED2, BAIAP2L2, C22orf23, MICALL1, ELFN2, C1QTNF6, ANKRD54, TMPRSS6, C22orf33, MIR658, MIR659, LOC100506241, or MIR4534 |
| chr22:46309501-47080249 | at least one of hsa-let-7b, PPARA, WNT7B, CELSR1, PKDREJ, GRAMD4, GTSE1, TTC38, C22orf26, TRMU, LOC150381, C22orf40, CN5H6.4, MIRLET7BHG, MIRLET7A3, MIRLET7B, LOC730668, LOC100271722, MIR3619, or MIR4763 |

Optionally, the gene set A includes at least one of CALML6, CCDC136, EGR1, FAM71E2, GLIS1, IFITM3, KNOP1, KRT76, KRT9, KRTAP10-10, MAF, MNS1, PROSER3, SCYL1, SLC16A6, SRC, TCEAL5, TMEM82, TRIM26, U2AF2, USP35, WBP2NL, WDR44, ZNF20, or ZNF700; and
the gene fragment set B includes at least one of chr2:179479501-179610249, chr2:207989501-208000249, chr2:219719501-219840249, chr2:3679501-3700249, chr3:126249501-126270249, chr3:129319501-129330249, chr3: 138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249, or chr22:46309501-47080249.

Optionally, a to-be-detected sample in the human tumor homologous recombination deficiency, tumor mutation burden and/or microsatellite instability grading detections is pan-cancer.

Optionally, tumor homologous recombination deficiency (HRD), tumor mutation burden (TMB) and/or microsatellite instability (MSI) grading and prediction are used for guidance in clinical diagnosis and treatment; and
optionally, the grading is divided into a high-grade group and a low-grade group.

Optionally, the product includes a primer, a probe, a reagent, a kit, a gene chip or a detection system used for detecting a gene type of a gene in the gene combination.

Optionally, the product performs a detection for an exon and related intron region of a gene in the gene set A and the gene fragment set B.

Optionally, a method for grading the human tumor homologous recombination deficiency, tumor mutation burden and/or microsatellite instability includes the following steps:
step S1: evaluating a gene mutation and a gene copy number variation of a gene included in the gene set A in tumor cell tissue, and evaluating a gene copy number variation in the gene fragment set B in the tumor cell tissue; and
step S2: judging whether the grading of the tumor homologous recombination deficiency (HRD), tumor mutation burden (TMB) and/or microsatellite instability (MSI) is high or low based on an evaluation result of step S1, and performing a prediction.

Optionally, the gene mutation includes a base substitution mutation, a deletion mutation, an insertion mutation and/or a fusion mutation, and the gene copy number variation includes increase of the gene copy number and/or decrease of the gene copy number.

Optionally, in step S1, by comparing sequencing data of the tumor cell tissue and normal tissue, the gene mutation and the copy number variation of the gene included in the gene set A are evaluated, and meanwhile, the gene copy number variation in the gene fragment set B is evaluated.

Optionally, in step S2, the tumor is graded as the high-grade group in a case that at least one gene in the gene set A has the gene mutation or the copy number variation, or at least one fragment in the gene fragment set B has increase of the gene copy number; otherwise, the tumor is graded as the low-grade group, namely, in a case that no gene in the gene set A has the gene mutation or the copy number variation, and meanwhile, no fragment in the gene fragment set B has increase of the gene copy number.

Optionally, any gene fragment is selected from the gene combination for combination to form a new gene combination, and the same method for grading the human tumor homologous recombination deficiency, tumor mutation burden and/or microsatellite instability is used for grading and predicting the tumor homologous recombination deficiency (HRD), tumor mutation burden (TMB) and microsatellite instability (MSI).

The technical solutions of the present application have the following advantages.
1. The detected gene combination in the present application is obtained from actual high-throughput sequencing data of Peking University First Hospital by specific pairwise clustering analysis. Data derived from the real world has higher reliability and credibility. Homologous recombination deficiency (HRD), tumor mutation burden (TMB), and microsatellite instability (MSI) grading and prediction can be performed accurately for the pan-cancer.
2. The gene combination in the present application has pluralism, and various gene combinations may be preferably selected for grading and predicting the pan-cancer homologous recombination deficiency (HRD), tumor mutation burden (TMB) and microsatellite instability (MSI) so as to be used for different clinical situations.
3. Compared with whole exome sequencing, the present application performs targeted sequencing analysis for a specific gene and a DNA fragment, a sequencing depth and accuracy may be improved remarkably on the premise of the same cost, cost may be reduced significantly on the premise of the same sequencing depth and accuracy, and universality is wide.

### BRIEF DESCRIPTION OF THE DRAWINGS

In order to more clearly describe specific implementations of the present application or technical solutions in the prior art, the accompanying drawings needed by the description in the specific implementations or in the prior art will be briefly introduced below. Apparently, the accompanying drawings in the following description are some implementations of the present application. Those ordinarily skilled in the art can also obtain other accompanying drawings according to these accompanying drawings without making creative work.
FIG 1 is a box plot of comparing a high-grade group with a low-grade group by calculating a value p through a Mann-Whitney U test after homologous recombination deficiency (HRD) grading is performed by using a gene combination of Example 1 of the present application in Experimental example 1 of the present application.
FIG 2 is a box plot of comparing a high-grade group with a low-grade group by calculating a value p through a Mann-Whitney U test after tumor mutation burden (TMB) grading is performed by using a gene combination of Example 1 of the present application in Experimental example 1 of the present application.
FIG 3 is a box plot of comparing a high-grade group with a low-grade group by calculating a value p through a Mann-Whitney U test after microsatellite instability (MSI) grading is performed by using a gene combination of Example 1 of the present application in Experimental example 1 of the present application.
FIG 4 is a box plot of comparing a high-grade group with a low-grade group by calculating a value p through a Mann-Whitney U test after homologous recombination deficiency (HRD) grading is performed by using a gene combination 1 in Experimental example 2 of the present application.
FIG 5 is a box plot of comparing a high-grade group with a low-grade group by calculating a value p through a Mann-Whitney U test after tumor mutation burden (TMB) grading is performed by using a gene combination 1 in Experimental example 2 of the present application.
FIG 6 is a box plot of comparing a high-grade group with a low-grade group by calculating a value p through a Mann-Whitney U test after microsatellite instability (MSI) grading is performed by using a gene combination 1 in Experimental example 2 of the present application.

### DETAILED DESCRIPTION

The following examples are provided for better further understanding the present application, which are not limited to the preferred implementations and do not constitute a limitation on the content and the protection scope of the present application, and any product the same as or similar to the present application obtained by anybody under the inspiration of the present application or by combining the present application with features in the other prior art falls within the protection scope of the present application.

Where specific experimental steps or conditions are not indicated in the examples can be performed according to operations of conventional experimental steps or conditions described in documents in the art. Whatever reagent or instrument used with no indicated manufacturer may be a conventional reagent product that is commercially available.

### Example 1 Gene combination (panel) for human tumor homologous recombination deficiency (HRD), tumor mutation burden (TMB) and microsatellite instability (MSI) grading

The inventor mainly uses an exon sequencing high-throughput database of patients in Peking University First Hospital for screening, and thus determines a gene combination (panel) for human tumor homologous recombination deficiency (HRD), tumor mutation burden (TMB) and microsatellite instability (MSI) grading. The gene combination includes a gene set A and a gene fragment set B.

The gene set A includes at least one of ASAH1, ASXL1, BCOR, BRAF, CALML6, CCDC136, CIDEC, COX18, CSF1R, CYP3A5, DEK, DNMT3A, EGR1, FAM71E2, FGFR1, FKBP7, FLT1, FLT3, FLT4, GNAQ, GLIS1, IDH2, IFITM3, IMMT, KDR, KIT, KMT2A, KNOP1, KRT76, KRT9, KRTAP10-10, KRTAP10-8, MAF, MECOM, MFRP, MLLT3, MNS1, MRTFA, MTOR, MYH11, NF1, NUP214, PDGFRA, PDGFRB, PML, PRB2, PROSER3, RAF1, RARA, RBM15, RET, REXO1, RPN1, RUNX1T1, SCYL1, SLC16A6, SRC, STAG2, TCEAL5, TET2, TMEM82, TP53, TRIM26, U2AF1, U2AF2, UGT1A1, USP35, VEGFA, WBP2NL, WDR44, ZNF20, ZNF700, or ZRSR2.

The gene fragment set B includes: chr2:179479501-179610249, chr2:207989501-208000249, chr2:219719501-219840249, chr2:3679501-3700249, chr3:126249501-126270249, chr3: 129319501-129330249, chr3: 138659501-138770249, chr3: 183999501-184020249, chr4: 1189501-1230249, chr4:8579501-8590249, chr4:9319501-9330249, chr5: 150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7: 100539501-100560249, chr8: 1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:63179501-63550249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249, ord chr22:46309501-47080249; and a position of a gene fragment in the gene fragment set B is annotated by using GRCh37 as a standard, numbers may change in GRCh38 or a new-version human reference genome that emerges in the future, but a position of a directional objective fragment and a gene available for detections do not change.

Optionally, genes included in the gene fragment set B in details are shown in the following table:

**Table 1 Gene fragment set B**

| Position of gene fragment | Genes available for detections |
|---|---|
| chr2:179479501-17961024 9 | at least one of TTN, MIR548N, or LOC100506866 |
| chr2:207989501-20800024 9 | KLF7 |
| chr2:219719501-21984024 9 | at least one of WNT6, CDK5R2, or WNT10A |
| chr2:3679501-3700249 | COLEC11 |
| chr3:126249501-12627024 9 | at least one of C3orf22 or CHST13 |
| chr3:129319501-12933024 9 | PLXND1 |
| chr3:138659501-13877024 9 | at least one of FOXL2, PRR23B, PRR23C, C3 orf72, or PRR23A |
| chr3:183999501-18402024 9 | at least one of PSMD2 or ECE2 |
| chr4:1189501-1230249 | at least one of CTBP1, SPON2, or LOC100130872 |
| chr4:8579501-8590249 | GPR78 |
| chr4:9319501-9330249 | at least one of LOC728369, LOC728373, LOC728379, USP17L5, LOC728393, LOC728400, or LOC728405 |
| chr5:150899501-15094024 9 | FAT2 |
| chr6:147819501-14784024 9 | SAMD5 |
| chr6:157089501-157110249 | at least one of ARID 1B or MIR4466 |
| chr6:164889501-16490024 9 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-10056024 9 | ACHE |
| chr8:1939501-1960249 | KBTBD11 |
| chr8:21999501-22070249 | at least one of BMP1, SFTPC, or LGI3 |
| chr8:29189501-29200249 | DUSP4 |
| chr9:91789501-91800249 | SHC3 |
| chr10:99419501-99440249 | at least one of PI4K2A or AVPI1 |
| chr11:17739501-17760249 | at least one of KCNC 1 or MYOD1 |
| chr11:63329501-63350249 | at least one of PLA2G16 or PLAAT2 |
| chr12:169501-250249 | at least one of IQSEC3 or LOC574538 |
| chr12:54329501-54350249 | at least one of HOXC12 or HOXC13 |
| chr12:63179501-63550249 | at least one of AVPR1A or PPM1H |
| chr12:7269501-7310249 | at least one of CLSTN3, RBP5, or MATL2963 |
| chr13:114519501-11453024 9 | GAS6 |
| chr15:73649501-73670249 | HCN4 |
| chr15:74209501-74220249 | at least one of LOXL1 or LOC100287616 |
| chr15:78409501-78430249 | CIB2 |
| chr15:83859501-83880249 | HDGFL3 |
| chr18:8809501-8820249 | MTCL1 |
| chr19:24059501-24070249 | ZNF726 |
| chr19:4229501-4250249 | at least one of EBI3 or CCDC94 |
| chr19:46879501-46900249 | PPP5C |
| chr20:22559501-22570249 | FOXA2 |
| chr20:62189501-62200249 | HELZ2 |
| chr21:45949501-46110249 | at least one of TSPEAR, KRTAP12-2, KRTAP12-1, KRTAP10-10, KRTAP10-4, KRTAP10-6, KRTAP10-7, KRTAP10-9, KRTAP10-1, KRTAP10-11, KRTAP10-2, KRTAP10-5, KRTAP10-8, KRTAP10-3, KRTAP12-3, or KRTAP12-4 |
| chr22:19499501-19760249 | at least one of GP1BB, SEPTIN5, TBX1, CLDN5, CDC45, LOC150185, or SEPT5-GP1BB |
| chr22:36649501-38700249 | at least one of hsa-mir-659, CSF2RB, CSNK1E, H1F0, IL2RB, LGALS1, LGALS2, MFNG, MPST, MYH9, NCF4, POLR2F, PVALB, RAC2, SOX10, SSTR3, TST, PLA2G6, GALR3, APOL1, EIF3D, PICK1, CACNG2, IFT27, TRIOBP, CDC42EP1, GCAT, SLC16A8, SH3BP1, MAFF, TXN2, TMEM184B, GGA1, CYTH4, CARD10, EIF3L, PDXP, NOL12, KCTD17, FOXRED2, BAIAP2L2, C22orf23, MICALL1, ELFN2, C1QTNF6, ANKRD54, TMPRSS6, C22orf33, MIR658, MIR659, LOC100506241, or MIR4534 |
| chr22:46309501-47080249 | at least one of hsa-let-7b, PPARA, WNT7B, CELSR1, PKDREJ, GRAMD4, GTSE1, TTC38, C22orf26, TRMU, LOC150381, C22orf40, CN5H6.4, MIRLET7BHG, MIRLET7A3, MIRLET7B, LOC730668, LOC100271722, MIR3619, or MIR4763 |

### Example 2 Method for grading and predicting human pan-cancer homologous recombination deficiency (HRD), tumor mutation burden (TMB) and microsatellite instability (MSI)

The present example provides a method for grading and detecting human tumor homologous recombination deficiency, tumor mutation burden and microsatellite instability, including performing grading and detecting the human pan-cancer homologous recombination deficiency (HRD), tumor mutation burden (TMB) and microsatellite instability (MSI) by using the gene combination (panel) in Example 1, detailed steps of which are as follows:
(1) pan-cancer (the pan-cancer here is defined as all types of cancer in TCGA pan-cancer data, including adrenal cancer, urothelium carcinoma, breast cancer, cervical cancer, bile duct cancer, colon cancer, lymphoma, esophagus cancer, glioblastoma, head and neck squamous cell carcinoma, renal chromophobe cell tumor, renal clear cell carcinoma, papillary renal cell carcinoma, leukemia, brain glioma, hepatocellular carcinoma, lung adenocarcinoma, squamous cell lung carcinoma, mesothelioma, ovarian serous cystadenocarcinoma, pancreatic cancer, pheochromocytoma and paraganglioma, prostatic cancer, rectal cancer, sarcoma, cutaneous melanoma, gastric cancer, testicular carcinoma, thyroid cancer, thymic carcinoma, endometrial cancer, uterine sarcoma, and uveal tract melanoma, and the pan-cancer mentioned in the present application is defined as this and is not repeated) tissue is taken and compared with a healthy control tissue specimen, and the pan-cancer tissue specimen may be a pan-cancer cell line, a fresh pan-cancer specimen, a frozen pan-cancer specimen or a paraffin-embedded pan-cancer specimen; and the healthy control tissue may be tissue of a known recognized person, or may also be para-carcinoma tissue of a patient suffering from the pan-cancer or other non-tumor tissue. In the present example, a paraffin-embedded pan-cancer specimen is selected, normal para-carcinoma tissue is used as the healthy control tissue, DNA is extracted through a conventional method, a library is established through a conventional method, finally targeted high-throughput sequencing is performed by using the gene combination (panel) in Example 1, sequencing data of the pan-cancer tissue and the healthy tissue is compared to obtain a situation of a gene mutation (Mutation) and a copy number variation (CNV) of each gene in a gene set A in a gene combination of the pan-cancer tissue as well as a situation of a copy number variation (CNV) of each gene in a gene fragment set B.
   The gene mutation includes a base substitution mutation, a deletion mutation, an insertion mutation and a fusion mutation, and the gene copy number variation includes increase of the gene copy number and decrease of the gene copy number.
(2) Judgment is performed based on the mutation and/or variation situation of each gene in the gene combination of the pan-cancer tissue obtained in step (1):
   the patient suffering from the pan-cancer is graded as a high-grade group in a case that at least one gene in the gene set A has the gene mutation or the copy number variation, or at least one region in the gene fragment set B has increase of the gene copy number, and has a higher homologous recombination deficiency (HRD) score, tumor mutation burden (TMB) and microsatellite instability (MSI) score; otherwise, the patient suffering from the pan-cancer is graded as as a low-grade group in a case that no gene in the gene set A has the gene mutation or the copy number variation, and meanwhile, no fragment in the gene fragment set B has increase of the gene copy number, and has a lower homologous recombination deficiency (HRD) score, tumor mutation burden (TMB) and microsatellite instability (MSI) score.

### Example 3

As an alternative implementation, in the present application, selecting and recombining genes in the gene combination (panel) in Example 1 are allowed to form a new gene combination, a judgment standard is that a gene mutation or a copy number variation of at least one gene of genes selected from the gene set A indicates that a patient suffering from the pan-cancer is graded as a high-grade group, or increase of a gene copy number of at least one region of gene fragments selected from the gene fragment set B indicates that the patient suffering from the pan-cancer is graded as the high-grade group; and otherwise, the patient suffering from the pan-cancer is graded as a low-grade group in a case that none of the genes selected from the gene set A has the mutation or the copy number variation, and meanwhile, none of the fragments selected from the gene fragment set B has increase of the copy number.

### Experimental example 1 Feasibility test for a gene combination and a detecting method for grading human tumor homologous recombination deficiency (HRD), tumor mutation burden (TMB) and microsatellite instability (MSI) in evaluation of human pan-cancer tumor homologous recombination deficiency (HRD), tumor mutation burden (TMB) and microsatellite instability (MSI) grading and prediction

A TCGA (PanCancer Atlas) pan-cancer database is a globally recognized pan-cancer database, which may be used for testing feasibility and credibility for evaluation of pan-cancer homologous recombination deficiency (HRD), tumor mutation burden (TMB) and microsatellite instability (MSI) grading and prediction in the present application.

TCGA (PanCancer Atlas) pan-cancer data has a total of 10967 pan-cancer cases, wherein 9896 cases have complete gene mutation and copy number variation data, which is applicable to an application condition of the present application.

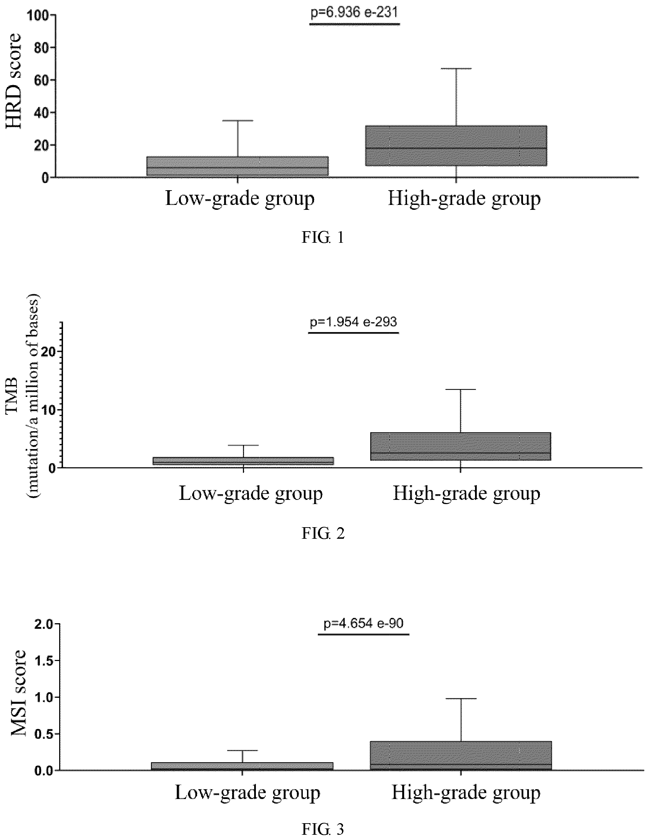

Implementation is performed according to the method of Example 2, the present experimental example selects all genes in the gene set A and all fragments in the gene fragment set B in Example 1 for implementation, and genes in the gene fragment set B actually used for detections are shown in the following Table 2. Homologous recombination deficiency (HRD), tumor mutation burden (TMB) and microsatellite instability (MSI) grading is performed on the above 9896 cases of patients, which is smoothly divided into a high-grade group and a low-grade group, wherein the high-grade group accounts for 77.0%, and the low-grade group accounts for 23.0%. According to the homologous recombination deficiency (HRD) score, tumor mutation burden (TMB) and microsatellite instability (MSI) score in the TCGA pan-cancer database, through a Mann-Whitney U test, it may be seen that the homologous recombination deficiency score (FIG 1), tumor mutation burden (FIG 2) and microsatellite instability score (FIG 3) of the high-grade group and the low-grade group have a statistic difference and are suitable for a grouping anticipation of the present application: the high-grade group has an obviously higher homologous recombination deficiency score (value p=6.936 e-231), tumor mutation burden (value p=1.954 e-293) and microsatellite instability score (value p=4.654 e-90). The homologous recombination deficiency (HRD) score greater than or equal to 42 is usually clinically defined as an HRD high-score group, if this standard is defined as an HRD golden standard for testing in the TCGA pan-cancer database, whether the high-grade group after classifying the homologous recombination deficiency (HRD) by using the gene combination of the present application is the HRD high-score group is judged, so sensitivity is 0.979, and a negative predictive value is 0.989. The tumor mutation burden (TMB) greater than or equal to 10/Mb is always clinically defined as a TMB high-score group, if this standard is defined as a TMB golden standard for testing in the TCGA pan-cancer database, whether a high-grade group after classifying the tumor mutation burden (TMB) by using the gene combination of the present application is the TMB high-score group is judged, so sensitivity is 0.989, and a negative predictive value is 0.994. The microsatellite instability (MSI) score greater than or equal to 10 is clinically defined as an MSI high-score group, if this standard is defined as an MSI golden standard for testing in the TCGA pan-cancer database, whether a high-grade group after classifying the microsatellite instability (MSI) by using the gene combination of the present application is the MSI high-score group is judged, so sensitivity is 0.973, and a negative predictive value is 0.996. Thus, using the gene combination of the present application for grading and predicting the homologous recombination deficiency (HRD), tumor mutation burden (TMB) and microsatellite instability (MSI) of the patient suffering from the pan-cancer is accurate and reliable.

**Table 2 Genes for actual detections for the gene fragment set B in Experimental example 1**

| Position of gene fragment | Genes available for detections in Experimental example |
|---|---|
| chr2:179479501-179610249 | TTN |
| chr2:207989501-208000249 | KLF7 |
| chr2:219719501-219840249 | WNT6 |
| chr2:3679501-3700249 | COLEC11 |
| chr3:126249501-126270249 | CHST13 |
| chr3:129319501-129330249 | PLXND1 |
| chr3:138659501-138770249 | FOXL2 |
| chr3:183999501-184020249 | PSMD2 |
| chr4:1189501-1230249 | CTBP1 |
| chr4:8579501-8590249 | GPR78 |
| chr4:9319501-9330249 | USP17L5 |
| chr5:150899501-150940249 | FAT2 |
| chr6:147819501-147840249 | SAMD5 |
| chr6:157089501-157110249 | ARID1B |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD11 |
| chr8:21999501-22070249 | BMP1 |
| chr8:29189501-29200249 | DUSP4 |
| chr9:91789501-91800249 | SHC3 |
| chr10:99419501-99440249 | PI4K2A |
| chr11:17739501-17760249 | MYOD1 |
| chr11:63329501-63350249 | PLAAT2 |
| chr12:169501-250249 | IQSEC3 |
| chr12:54329501-54350249 | HOXC13 |
| chr12:63179501-63550249 | AVPR1A |
| chr12:7269501-7310249 | CLSTN3 |
| chr13:114519501-114530249 | GAS6 |
| chr15:73649501-73670249 | HCN4 |
| chr15:74209501-74220249 | LOXL1 |
| chr15:78409501-78430249 | CIB2 |
| chr15:83859501-83880249 | HDGFL3 |
| chr18:8809501-8820249 | MTCL1 |
| chr19:24059501-24070249 | ZNF726 |
| chr19:4229501-4250249 | EBI3 |
| chr19:46879501-46900249 | PPP5C |
| chr20:22559501-22570249 | FOXA2 |
| chr20:62189501-62200249 | HELZ2 |
| chr21:45949501-46110249 | TSPEAR |
| chr22:19499501-19760249 | SEPTIN5 |
| chr22:36649501-38700249 | MYH9 |
| chr22:46309501-47080249 | WNT7B |

### Experimental example 2 Feasibility test for a preferred gene combination and detecting method in evaluation of human pan-cancer homologous recombination deficiency (HRD), tumor mutation burden (TMB) and microsatellite instability (MSI) grading and prediction

The present application allows to select any gene fragment from the gene combination (panel) for combination to form a new gene combination, and the same judgment standard is used for grading and predicting the pan-cancer homologous recombination deficiency (HRD), tumor mutation burden (TMB) and microsatellite instability (MSI). Here, a gene set A1 (see Table 3) selected from the gene set A of the gene combination (panel) and a gene fragment set B1 (see Table 4) selected from the gene fragment set B constitute a gene combination 1 (panel 1) for grading and predicting the pan-cancer homologous recombination deficiency (HRD), tumor mutation burden (TMB) and microsatellite instability (MSI), and a feasibility analysis is performed by using the TCGA (PanCancer Atlas) pan-cancer database. Likewise, a judgment standard is that in a case that at least one gene of the gene set A1 has a gene mutation or a copy number variation, or at least one region in the gene fragment set B1 has increase of the gene copy number, it indicates that the patient suffering from the pan-cancer is graded as the high-grade group and has a higher homologous recombination deficiency (HRD) score, tumor mutation burden (TMB) and microsatellite instability (MSI) score; and otherwise, this type of patient suffering from the pan-cancer is graded as the low-grade group in a case that no gene in the gene set A1 has the gene mutation or the copy number variation, and meanwhile, no fragment in the gene fragment set B1 has increase of the gene copy number, and has a lower homologous recombination deficiency (HRD) score, tumor mutation burden (TMB) and microsatellite instability (MSI) score. It needs to be noted specially that in the present experimental example, the gene combination 1 (panel 1) is preferably selected on the basis of the gene combination (panel), and has lower cost due to its fewer target spots.

**Table 3 Gene set A1**

| Name of gene |
|---|
| CALML6, CCDC136, EGR1, FAM71E2, GLIS1, IFITM3, KNOP1, KRT76, KRT9, KRTAP10-10, MAF, MNS1, PROSER3, SCYL1, SLC16A6, SRC, TCEAL5, TMEM82, TRIM26, U2AF2, USP35, WBP2NL, WDR44, ZNF20, and ZNF700 |

**Table 4 Gene fragment set B1**

| Position of gene fragment | Genes available for detections in Experimental example |
|---|---|
| chr2:179479501-179610249 | TTN |
| chr2:207989501-208000249 | KLF7 |
| chr2:219719501-219840249 | WNT6 |
| chr2:3679501-3700249 | COLEC11 |
| chr3:126249501-126270249 | CHST13 |
| chr3:129319501-129330249 | PLXND1 |
| chr3:138659501-138770249 | FOXL2 |
| chr3:183999501-184020249 | PSMD2 |
| chr4:1189501-1230249 | CTBP1 |
| chr4:8579501-8590249 | GPR78 |
| chr4:9319501-9330249 | USP17L5 |
| chr5:150899501-150940249 | FAT2 |
| chr6:147819501-147840249 | SAMD5 |
| chr6:157089501-157110249 | ARID1B |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD11 |
| chr8:21999501-22070249 | BMP1 |
| chr8:29189501-29200249 | DUSP4 |
| chr9:91789501-91800249 | SHC3 |
| chr10:99419501-99440249 | PI4K2A |
| chr11:17739501-17760249 | MYOD1 |
| chr11:63329501-63350249 | PLAAT2 |
| chr12:169501-250249 | IQSEC3 |
| chr12:54329501-54350249 | HOXC13 |
| chr12:7269501-7310249 | CLSTN3 |
| chr13:114519501-114530249 | GAS6 |
| chr15:73649501-73670249 | HCN4 |
| chr15:74209501-74220249 | LOXL1 |
| chr15:78409501-78430249 | CIB2 |
| chr15:83859501-83880249 | HDGFL3 |
| chr18:8809501-8820249 | MTCL1 |
| chr19:24059501-24070249 | ZNF726 |
| chr19:4229501-4250249 | EBI3 |
| chr19:46879501-46900249 | PPP5C |
| chr20:22559501-22570249 | FOXA2 |
| chr20:62189501-62200249 | HELZ2 |
| chr21:45949501-46110249 | TSPEAR |
| chr22:19499501-19760249 | SEPTIN5 |
| chr22:36649501-38700249 | MYH9 |
| chr22:46309501-47080249 | WNT7B |

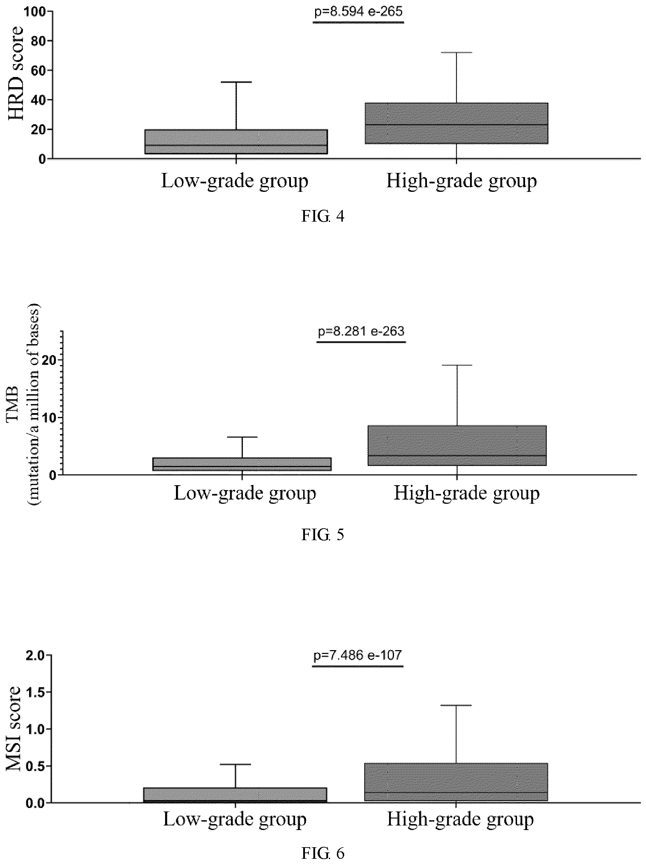

Implementation is performed according to the method of Example 2, the gene combination 1 (panel 1) performs grading of the homologous recombination deficiency (HRD), tumor mutation burden (TMB) and microsatellite instability (MSI) on the above 9896 cases of patients in the pan-cancer database, which is smoothly divided into a high-grade group and a low-grade group, wherein the high-grade group accounts for 39.1%, and the low-grade group accounts for 60.9%. According to the homologous recombination deficiency (HRD) score, tumor mutation burden (TMB) and microsatellite instability (MSI) score in the TCGA pan-cancer database, through a Mann-Whitney U test, it may be seen that the homologous recombination deficiency score (FIG 4), tumor mutation burden (FIG 5) and microsatellite instability score (FIG 6) of the high-grade group and the low-grade group have a statistic difference and are suitable for a grouping anticipation of the present application: the high-grade group has a obviously higher homologous recombination deficiency score (value p=8.594 e-265), tumor mutation burden (value p=8.281 e-263) and microsatellite instability score (value p=7.486 e-107). The homologous recombination deficiency (HRD) score greater than or equal to 42 is usually clinically defined as an HRD high-score group, if this standard is defined as an HRD golden standard for testing in the TCGA pan-cancer database, whether the high-grade group after classifying the homologous recombination deficiency (HRD) by using the gene combination 1 of the present application is the HRD high-score group is judged, so sensitivity is 0.712, and a negative predictive value is 0.944. The tumor mutation burden (TMB) greater than or equal to 10/Mb is always clinically defined as a TMB high-score group, if this standard is defined as a TMB golden standard for testing in the TCGA pan-cancer database, whether a high-grade group after classifying the tumor mutation burden (TMB) by using the gene combination 1 of the present application is the TMB high-score group is judged, so sensitivity is 0.692, and a negative predictive value is 0.935. The microsatellite instability (MSI) score greater than or equal to 10 is clinically defined as an MSI high-score group, if this standard is defined as an MSI golden standard for testing in the TCGA pan-cancer database, whether a high-grade group after classifying the microsatellite instability (MSI) by using the gene combination 1 of the present application is the MSI high-score group is judged, so sensitivity is 0.704, and a negative predictive value is 0.985. Thus, another gene combination selected from the gene combination (panel) of the present application may still be used for grading and predicting the homologous recombination deficiency (HRD), tumor mutation burden (TMB) and microsatellite instability (MSI) of the patient suffering from the pan-cancer.

Apparently, the above examples are merely examples for clear description, but not for limiting the implementations. Those ordinarily skilled in the art can also make modifications or variations in other different forms based on the above description. All implementations do not need to be and cannot be exhaustively cited here. Apparent modifications or variations derived from this still fall within the protection scope of the present disclosure.

## Claims

1. Use of a gene combination in the preparation of a product for human tumor homologous recombination deficiency, tumor mutation burden and/or microsatellite instability grading detections, wherein the gene combination consists of a gene set A and a gene fragment set B;
the gene set A comprises at least one of ASAH1, ASXL1, BCOR, BRAF, CALML6, CCDC136, CIDEC, COX18, CSF1R, CYP3A5, DEK, DNMT3A, EGR1, FAM71E2, FGFR1, FKBP7, FLT1, FLT3, FLT4, GLIS1, GNAQ, IDH2, IFITM3, IMMT, KDR, KIT, KMT2A, KNOP1, KRT76, KRT9, KRTAP10-10, KRTAP10-8, MAF, MECOM, MFRP, MLLT3, MNS1, MRTFA, MTOR, MYH11, NF1, NUP214, PDGFRA, PDGFRB, PML, PRB2, PROSER3, RAF1, RARA, RBM15, RET, REXO1, RPN1, RUNX1T1, SCYL1, SLC16A6, SRC, STAG2, TCEAL5, TET2, TMEM82, TP53, TRIM26, U2AF1, U2AF2, UGT1A1, USP35, VEGFA, WBP2NL, WDR44, ZNF20, ZNF700 and ZRSR2;
the gene fragment set B comprises at least one of chr2:179479501-179610249, chr2:207989501-208000249, chr2:219719501-219840249, chr2:3679501-3700249, chr3:126249501-126270249, chr3:129319501-129330249, chr3:138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:63179501-63550249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249 and chr22:46309501-47080249; and a position of a gene fragment in the gene fragment set B is annotated by using GRCh37 as a standard.

2. The use according to claim 1, wherein genes comprised in the gene fragment set B in details are in the following table:
**Table 1 Gene fragment set B**
| Position of gene fragment | Genes available for detections |
|---|---|
| chr2:179479501-179610249 | at least one of TTN, MIR548N, and LOC100506866 |
| chr2:207989501-208000249 | KLF7 |
| chr2:219719501-219840249 | at least one of WNT6, CDK5R2, and WNT10A |
| chr2:3679501-3700249 | COLEC 11 |
| chr3:126249501-126270249 | at least one of C3orf22 and CHST13 |
| chr3:129319501-129330249 | PLXND1 |
| chr3: 138659501-138770249 | at least one of FOXL2, PRR23B, PRR23C, C3orf72, and PRR23A |
| chr3:183999501-184020249 | at least one of PSMD2 and ECE2 |
| chr4:1189501-1230249 | at least one of CTBP1, SPON2, and LOC100130872 |
| chr4:8579501-8590249 | GPR78 |
| chr4:9319501-9330249 | at least one of LOC728369, LOC728373, LOC728379, USP17L5, LOC728393, LOC728400, and LOC728405 |
| chr5:150899501-150940249 | FAT2 |
| chr6:147819501-147840249 | SAMD5 |
| chr6:157089501-157110249 | at least one of ARID1B and MIR4466 |
| chr6:164889501-164900249 | C6orf118 |
| chr6:20399501-20410249 | E2F3 |
| chr6:26519501-26530249 | HCG11 |
| chr6:71659501-71670249 | B3GAT2 |
| chr6:73329501-73340249 | KCNQ5 |
| chr7:100539501-100560249 | ACHE |
| chr8:1939501-1960249 | KBTBD11 |
| chr8:21999501-22070249 | at least one of BMP1, SFTPC, and LGI3 |
| chr8:29189501-29200249 | DUSP4 |
| chr9:91789501-91800249 | SHC3 |
| chr10:99419501-99440249 | at least one of PI4K2A and AVPI1 |
| chr11:17739501-17760249 | at least one of KCNC1 and MYOD1 |
| chr11:63329501-63350249 | at least one of PLA2G16 and PLAAT2 |
| chr12:169501-250249 | at least one of IQSEC3 and LOC574538 |
| chr12:54329501-54350249 | at least one of HOXC12 and HOXC13 |
| chr12:63179501-63550249 | at least one of AVPR1A and PPM1H |
| chr12:7269501-7310249 | at least one of CLSTN3, RBP5, and MATL2963 |
| chr13:114519501-114530249 | GAS6 |
| chr15:73649501-73670249 | HCN4 |
| chr15:74209501-74220249 | at least one of LOXL1 and LOC100287616 |
| chr15:78409501-78430249 | CIB2 |
| chr15:83859501-83880249 | HDGFL3 |
| chr18:8809501-8820249 | MTCL1 |
| chr19:24059501-24070249 | ZNF726 |
| chr19:4229501-4250249 | at least one of EBI3 and CCDC94 |
| chr19:46879501-46900249 | PPP5C |
| chr20:22559501-22570249 | FOXA2 |
| chr20:62189501-62200249 | HELZ2 |
| chr21:45949501-46110249 | at least one of TSPEAR, KRTAP12-2, KRTAP12-1, KRTAP10-10, KRTAP10-4, KRTAP10-6, KRTAP10-7, KRTAP10-9, KRTAP10-1, KRTAP10-11, KRTAP10-2, KRTAP10-5, KRTAP10-8, KRTAP10-3, KRTAP12-3, and KRTAP12-4 |
| chr22:19499501-19760249 | at least one of GP1BB, SEPTIN5, TBX1, CLDN5, CDC45, LOC150185, and SEPT5-GP1BB |
| chr22:36649501-38700249 | at least one of hsa-mir-659, CSF2RB, CSNK1E, H1F0, IL2RB, LGALS1, LGALS2, MFNG, MPST, MYH9, NCF4, POLR2F, PVALB, RAC2, SOX10, SSTR3, TST, PLA2G6, GALR3, APOL1, EIF3D, PICK1, CACNG2, IFT27, TRIOBP, CDC42EP1, GCAT, SLC16A8, SH3BP1, MAFF, TXN2, TMEM184B, GGA1, CYTH4, CARD10, EIF3L, PDXP, NOL12, KCTD17, FOXRED2, BAIAP2L2, C22orf23, MICALL1, ELFN2, C1QTNF6, ANKRD54, TMPRSS6, C22orf33, MIR658, MIR659, LOC100506241, and MIR4534 |
| chr22:46309501-47080249 | at least one of hsa-let-7b, PPARA, WNT7B, CELSR1, PKDREJ, GRAMD4, GTSE1, TTC38, C22orf26, TRMU, LOC150381, C22orf40, CN5H6.4, MIRLET7BHG, MIRLET7A3, MIRLET7B, LOC730668, LOC100271722, MIR3619, and MIR4763 |
optionally, the gene set A comprises at least one of CALML6, CCDC136, EGR1, FAM71E2, GLIS1, IFITM3, KNOP1, KRT76, KRT9, KRTAP10-10, MAF, MNS1, PROSER3, SCYL1, SLC16A6, SRC, TCEAL5, TMEM82, TRIM26, U2AF2, USP35, WBP2NL, WDR44, ZNF20 and ZNF700; and
the gene fragment set B comprises at least one of chr2:179479501-179610249, chr2:207989501-208000249, chr2:219719501-219840249, chr2:3679501-3700249, chr3:126249501-126270249, chr3:129319501-129330249, chr3:138659501-138770249, chr3:183999501-184020249, chr4:1189501-1230249, chr4:8579501-8590249, chr4:9319501-9330249, chr5:150899501-150940249, chr6:147819501-147840249, chr6:157089501-157110249, chr6:164889501-164900249, chr6:20399501-20410249, chr6:26519501-26530249, chr6:71659501-71670249, chr6:73329501-73340249, chr7:100539501-100560249, chr8:1939501-1960249, chr8:21999501-22070249, chr8:29189501-29200249, chr9:91789501-91800249, chr10:99419501-99440249, chr11:17739501-17760249, chr11:63329501-63350249, chr12:169501-250249, chr12:54329501-54350249, chr12:7269501-7310249, chr13:114519501-114530249, chr15:73649501-73670249, chr15:74209501-74220249, chr15:78409501-78430249, chr15:83859501-83880249, chr18:8809501-8820249, chr19:24059501-24070249, chr19:4229501-4250249, chr19:46879501-46900249, chr20:22559501-22570249, chr20:62189501-62200249, chr21:45949501-46110249, chr22:19499501-19760249, chr22:36649501-38700249 and chr22:46309501-47080249.

3. The use according to claim 1 or 2, wherein a to-be-detected sample in the human tumor homologous recombination deficiency, tumor mutation burden and/or microsatellite instability grading detections is pan-cancer.

4. The use according to claim 3, wherein human tumor homologous recombination deficiency, tumor mutation burden and/or microsatellite instability grading and prediction are used for guidance in clinical diagnosis and treatment; and
optionally, the grading is divided into a high-grade group and a low-grade group.

5. The use according to any one of claims 1-4, wherein the product comprises a primer, a probe, a reagent, a kit, a gene chip or a detection system used for detecting a gene type of a gene in the gene combination.

6. The use according to claim 5, wherein the product performs a detection for an exon and related intron region of a gene in the gene set A and the gene fragment set B.

7. The use according to any one of claims 1-6, wherein a method for grading the human tumor homologous recombination deficiency, the tumor mutation burden and/or microsatellite instability comprises the following steps:
step S1: evaluating a gene mutation and a gene copy number variation of a gene comprised in the gene set A in tumor cell tissue, and evaluating a gene copy number variation in the gene fragment set B in the tumor cell tissue; and
step S2: judging whether the grading of tumor homologous recombination deficiency, tumor mutation burden and/or microsatellite instability is high or low based on an evaluation result of step S1, and performing a prediction.

8. The use according to claim 7, wherein the gene mutation comprises a base substitution mutation, a deletion mutation, an insertion mutation and/or a fusion mutation, and the gene copy number variation comprises increase of the gene copy number and/or decrease of the gene copy number.

9. The use according to claim 7 or 8, wherein in step S1, by comparing sequencing data of the tumor cell tissue and normal tissue, the gene mutation and the copy number variation of the gene comprised in the gene set A are evaluated, and meanwhile, the gene copy number variation in the gene fragment set B is evaluated.

10. The use according to any one of claims 7-9, wherein in step S2, the tumor is graded as the high-grade group in a case that at least one gene in the gene set A has the gene mutation or the copy number variation, or at least one fragment in the gene fragment set B has increase of the gene copy number; otherwise, the tumor is graded as the low-grade group, namely, in a case that no gene in the gene set A has the gene mutation or the copy number variation, and meanwhile, no fragment in the gene fragment set B has increase of the gene copy number.

11. The use according to any one of claims 1-10, wherein any gene fragment is selected from the gene combination for combination to form a new gene combination, and the same grading method for the human tumor homologous recombination deficiency, the tumor mutation burden and/or the microsatellite instability is used for grading and predicting the tumor homologous recombination deficiency, the tumor mutation burden and the microsatellite instability, so as to guide clinical diagnosis and treatment.
